# EUROPEAN PATENT APPLICATION

(11) **EP 4 293 004 A1**
(43) Date of publication of application: **20.12.2023**
(21) Application number: 22752630.8
(22) Date of filing: 31.01.2022
(51) Int. Cl.: C07C 15/20, C07B 61/00, C07F 7/08, C01B 32/15, C01B 32/184

(54) **NOVEL GRAPHENE NANORIBBON AND METHOD FOR PRODUCING SAME**

(30) Priority: 10.02.2021 JP 2021020183
(71) Applicant: National University Corporation Tokai National Higher Education and Research System, Nagoya-shi, Aichi 464-8601 (JP); Taoka Chemical Co., Ltd., Yodogawa-ku Osaka-shi Osaka 532-0006 (JP)
(72) Inventor: ITAMI, Kenichiro, Nagoya-shi, Aichi 464-8601 (JP); ITO, Hideto, Nagoya-shi, Aichi 464-8601 (JP); MATSUSHIMA, Kaho, Nagoya-shi, Aichi 464-8601 (JP); NAKATSUJI, Hidefumi, Osaka-shi, Osaka 532-0006 (JP); UENO, Ryoma, Osaka-shi, Osaka 532-0006 (JP); ISHIDA, Shunsuke, Osaka-shi, Osaka 532-0006 (JP); OKUDA, Sachie, Osaka-shi, Osaka 532-0006 (JP)
(74) Representative: Gevers & Orès
(86) International application number: PCT/JP2022/003669
(87) International publication number: WO 2022/172801

(57) **Abstract**

A graphene nanoribbon represented by formula (1): 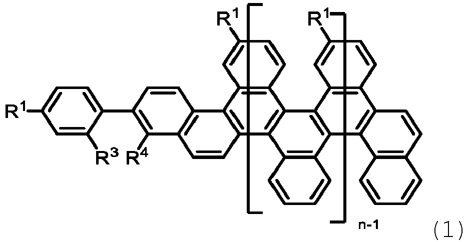 wherein R¹ represents a linear alkyl group having 1 to 12 carbon atoms, R³ and R⁴ are both hydrogen atoms, or R³ and R⁴ taken together form a group represented by -SiR^{2a}R^{2b}-, wherein R^{2a} and R^{2b} are the same or different, and each represents a hydrogen atom, an optionally branched alkyl group having 1 to 4 carbon atoms, or a phenyl group, and n represents an integer of 1 or more, is a novel GNR obtained by a simpler and industrially advantageous method for GNRs.

## Description

### Technical Field

The present invention relates to an improved method for producing a graphene nanoribbon and a novel graphene nanoribbon obtained by the production method.

### Background Art

Graphene nanoribbons (sometimes referred to below as "GNRs") refer to a substance that shows promise in application to semiconductors, solar batteries, transparent electrodes, highspeed transistors, organic EL devices, and the like. Production methods for the GNRs are broadly divided into two methods: a top-down method and a bottom-up method. In particular, the latter is attractive in its ability to synthesize a large amount of GNRs with precise control of the edge structure and width.

The present inventors focused on the bottom-up method and conducted extensive research on its production method. As a result, as a method for producing GNRs comprising fewer steps and involving reduced side reactions, the inventors found a method for polymerizing a silole compound by using an alkyne compound etc. having a specific structure as an initiator (see, for example, Patent Literature (PTL) 1).

### Citation List

### Patent Literature

PTL 1: WO2020/184625

### Summary of Invention

### Technical Problem

However, further improvement is required for the method of PTL 1 in terms of industrial practice, and a simpler production method for GNRs is in demand.

Accordingly, an object of the present invention is to provide a simpler and industrially advantageous production method for a GNR, and novel GNRs obtained by the production method.

### Solution to Problem

The present inventors conducted extensive research to achieve the object, and found that the following method is capable of producing GNRs in a simpler and industrially advantageous manner, and providing the novel GNRs described below. Specifically, the invention encompasses the following embodiments.

[1] A graphene nanoribbon represented by formula (1): 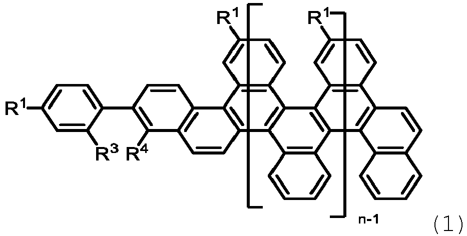 wherein R¹ represents a linear alkyl group having 1 to 12 carbon atoms, R³ and R⁴ are both hydrogen atoms, or R³ and R⁴ taken together form a group represented by -SiR^{2a}R^{2b}-, wherein R^{2a} and R^{2b} are the same or different, and each represents a hydrogen atom, an optionally branched alkyl group having 1 to 4 carbon atoms, or a phenyl group, and n represents an integer of 1 or more.
[2] The graphene nanoribbon according to [1], which is represented by formula (1-1): 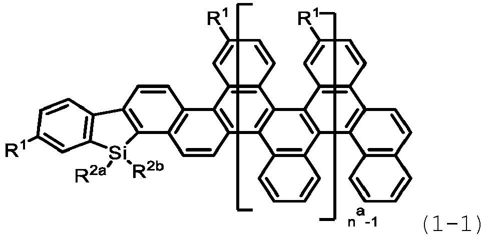
   wherein R¹, R^{2a}, and R^{2b} are as defined above, and n^{a} represents an integer of 1 or more; and/or
   formula (1-2): 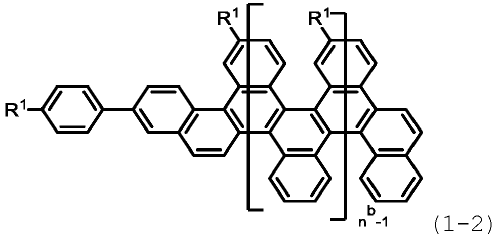
   wherein R¹ is as defined above, and n^{b} represents an integer of 1 or more.
[3] A method for producing the graphene nanoribbon of [1] or [2], comprising
   polymerizing a silole compound represented by formula (2): 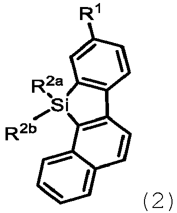
   wherein R¹, R^{2a}, and R^{2b} are as defined above,
   in the presence of 0.01 to 0.4 mol, based on 1 mole of silole compound represented by formula (2), of a palladium compound, o-chloranil, and a silver compound.
[4] A graphene nanoribbon obtained by polymerizing a silole compound represented by formula (2): 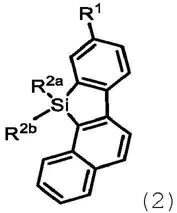
   wherein R¹ represents a linear alkyl group having 1 to 12 carbon atoms, and R^{2a} and R^{2b} each represent an optionally branched alkyl group having 1 to 4 carbon atoms, or a phenyl group,
   in the presence of 0.01 to 0.4 mol, based on 1 mole of silole compound represented by formula (2), of a palladium compound, o-chloranil, and a silver compound.
[5] A silole compound represented by formula (3): 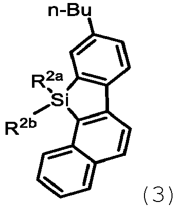 wherein n-Bu represents an n-butyl group, and R^{2a} and R^{2b} are the same or different, and each represents an optionally branched alkyl group having 1 to 4 carbon atoms, or a phenyl group.

### Advantageous Effects of Invention

According to the present invention, GNRs can be produced under specific conditions described above without using an alkyne compound represented by the following formula (4A): 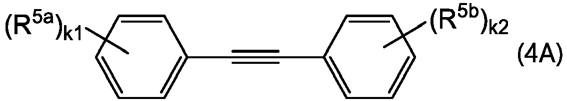 (wherein
R^{5a} and R^{5b} are the same or different, and each represents a hydrogen atom, a halogen atom, an alkyl group, a cycloalkyl group, a (poly)ether group, an ester group, a boronic acid or an ester group thereof, a monovalent aromatic hydrocarbon group, or a monovalent heterocyclic group, and
k1 and k2 are the same or different, and each represents an integer of 1 to 3, wherein when k1 and k2 are an integer of 2 or more, each R^{3a} and/or R^{3b} is optionally the same or different),
a K region-containing aromatic compound represented by the following formula (4B): 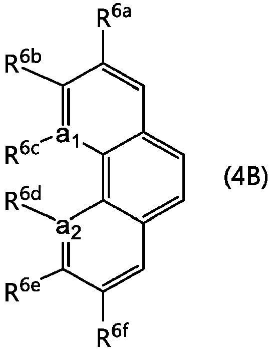
(wherein R^{6a}, R^{6b}, R^{6c}, R^{6d}, R^{6e}, and R^{6f} are the same or different, and each represents a hydrogen atom, a halogen atom, an alkyl group, a cycloalkyl group, a (poly)ether group, an ester group, a boronic acid or an ester group thereof, a monovalent aromatic hydrocarbon group, or a monovalent heterocyclic group, and a₁ and a₂ are the same or different, and each represents a carbon atom or a nitrogen atoms),
dibenzocyclooctadiyne, benzothiophene, or benzofuran (sometimes referred to below as "initiator compounds"), which have so far been considered essential to initiate the polymerization reaction. Accordingly, since additional use of an initiator compound is not required, GNRs can be produced more inexpensively and simply. Furthermore, novel GNRs that do not contain a unit derived from the initiator compound and that could not be produced by previously known production methods, i.e., a GNR represented by formula (1), can be produced.

According to previously known production methods, it was considered necessary to use an equivalent amount or more of a palladium compound based on the silole compound. However, according to the method for producing a GNR of the present invention, GNRs can be produced using a catalytic amount (i.e., less than the equivalent amount based on the silole compound) of a palladium compound. Additionally, the amount of a silver compound for use can also be reduced; thus, according to the method for producing a GNR of the present invention, GNRs can be produced more inexpensively.

### Brief Description of Drawings

Fig. 1 is a ¹H-NMR spectrum of the silole compound obtained in Example 1.
Fig. 2 is a ¹³C-NMR spectrum of the silole compound obtained in Example 1.

### Description of Embodiments

In the present specification, the terms "comprise" and "contain" include the concepts of consisting essentially of and consisting of.

In the present specification, the range represented by "A to B" means A or more and B or less unless otherwise specified.

### (1) Method for Producing GNR of the Present Invention

The method for producing a GNR of the present invention is characterized by polymerizing a silole compound having a specific structure represented by formula (2): 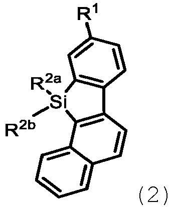
wherein R¹ represents a linear alkyl group having 1 to 12 carbon atoms, and R^{2a} and R^{2b} are the same or different, and each represents a hydrogen atom, an optionally branched alkyl group having 1 to 4 carbon atoms, or a phenyl group,
in the presence of 0.01 to 0.4 mol, based on 1 mole of silole compound represented by formula (2), of a palladium compound, o-chloranil, and a silver compound.

### (1-1) Silole Compound

The silole compound for use in the present invention has a structure represented by formula (2) above. In formula (2) above, substituent R¹ must be a linear alkyl group having 1 to 12 carbon atoms. If substituent R¹ is a hydrogen atom or a branched alkyl group, the polymerization reaction hardly proceeds and a GNR with a relatively higher molecular weight (i.e., a weight average molecular weight (Mw) measured in terms of polystyrene by size exclusion chromatography (sometimes referred to below as "SEC") under the conditions described in the Examples section below of, for example, 3000 or more) cannot be obtained. The linear alkyl group having 1 to 12 carbon atoms is preferably a linear alkyl group having 2 to 8 carbon atoms, and particularly preferably a linear alkyl group having 4 carbon atoms (an n-butyl group) . Substituents R^{2a} and R^{2b} are each a hydrogen atom, an optionally branched alkyl group having 1 to 4 carbon atoms, or a phenyl group. Examples of the optionally branched alkyl group include methyl, ethyl, n-propyl, isopropyl, n-butyl sec-butyl, and tert-butyl. From the viewpoint of ease of production of the silole compound represented by formula (2) above, R^{2a} and R^{2b} are each preferably a methyl group, an ethyl group, or a phenyl group, and R^{2a} and R^{2b} are preferably the same substituents. These silole compounds can be used alone or in a combination of two or more.

The silole compound represented by formula (2) above can be produced in accordance with known methods (e.g., the method described in PTL 1). Specifically, as in the method described in the Examples section below, the silole compound can be produced by reacting a compound in which R¹ is a halogen atom (e.g., a chlorine atom, bromine atom, or iodine atom) (e.g., compound S23 in Synthesis Example 5 of PTL 1) instead of a linear alkyl group having 1 to 12 carbon atoms with a Grignard reagent provided with a linear alkyl group having 1 to 12 carbon atoms in the presence of an iron compound, in accordance with the method in Synthesis Example 6 in PTL 1.

### (1-2) Palladium Compound

In the present invention, any palladium compounds, known as synthetic catalysts for polymer compounds, can be used. Of these, divalent palladium compounds are preferred. Examples of usable palladium compounds include Pd(OH)₂, Pd(OCOCH₃)₂, Pd₂(dba)₃, Pd(OCOCF₃)₂, Pd(acac)₂, PdCl₂, PdBr₂, PdI₂, Pd(NO₃)₂, and Pd(CH₃CN)₄(SbF₆)₂. "acac" means acetylacetonate, and "dba" means dibenzylideneacetone. The use of a weakly cationic palladium compound makes it less likely to disintegrate the silole skeleton of the substrate, and makes it more likely to obtain a higher-molecular-weight GNR. In the present invention, from such viewpoints, Pd(OH)₂, Pd(OCOCH₃)₂, Pd(OCOCF₃)₂, PdBr₂, PdI₂, and Pd(CH₃CN)₄(SbF₆)₂ are preferred, Pd(OCOCH₃)₂, Pd(OCOCF₃)₂, PdBr₂, PdI₂, and Pd(CH₃CN)₄(SbF₆)₂ are more preferred, and Pd(OCOCH₃)₂ is particularly preferred. These palladium compounds can be used alone or in a combination of two or more.

The amount of the palladium compound for use is 0.01 to 0.4 mol, and preferably 0.05 to 0.3 mol, per 1 mole of the silole compound. If the amount of the palladium compound for use is less than 0.01 mol or exceeds 0.4 mol, then a GNR with a relatively higher molecular weight cannot be obtained.

### (1-3) o-Chloranil

In the present invention, the amount of o-chloranil for use may be any amount. From the viewpoint of easily obtaining a GNR with a relatively higher molecular weight, the amount is, for example, 0.5 to 5.0 mol, preferably 1.0 to 3.0 mol, and more preferably 1.5 to 2.5 mol, per 1 mole of the silole compound. If o-chloranil is not used, the polymerization reaction hardly proceeds, and the GNR of the present invention cannot be obtained.

### (1-4) Silver Compound

The silver compound for use in the present invention may be any silver compound, and examples include organic silver compounds, such as silver acetate, silver pivalate (AgOPiv), silver trifluoromethanesulfonate (AgOTf), and silver benzoate (AgOCOPh); and inorganic silver compounds, such as silver nitrate, silver fluoride, silver chloride, silver bromide, silver iodide, silver sulfate, silver oxide, silver sulfide, silver tetrafluoroborate (AgBF₄), silver hexafluorophosphate (AgPF₆), and silver hexafluoroantimonate (AgSbF₆). In the present invention, from the viewpoint of easily obtaining a higher-molecular-weight GNR, inorganic silver compounds are preferred; silver tetrafluoroborate (AgBF₄), silver hexafluorophosphate (AgPF₆), silver hexafluoroantimonate (AgSbF₆), etc. are more preferred; silver tetrafluoroborate (AgBF₄), silver hexafluoroantimonate (AgSbF₆)etc. are even more preferred; and silver hexafluoroantimonate (AgSbF₆)is particularly preferred. These silver compounds can be used alone or in a combination of two or more.

The amount of the silver compound for use is, for example, 0.1 to 3.0 mol per 1 mole of the palladium compound. From the viewpoint of easily obtaining a GNR with a relatively higher molecular weight, the amount is preferably 0.2 to 1.5 mol, and more preferably 0.3 to 1.3 mol, per 1 mole of the palladium compound.

### (1-5) Others

The present invention is preferably performed in a solvent. Examples of usable solvents include aliphatic hydrocarbons, such as pentane, hexane, heptane, and cyclohexane; halogenated aliphatic hydrocarbons, such as dichloromethane, dichloroethane (DCE), chloroform (CHCl₃), carbon tetrachloride, and trichloroethylene (TCE); and halogenated aromatic hydrocarbons, such as monochlorobenzene (PhCl), dichlorobenzene (PhCl₂) bromobenzene (PhBr), and 1,3,5-tribromobenzene (PhBr₃). These solvents can be used alone or in a combination of two or more. Of these solvents, halogenated aliphatic hydrocarbons and halogenated aromatic hydrocarbons are preferred, and monochlorobenzene (PhCl) and dichlorobenzene (PhCl₂) are more preferred. In addition to the above components, additives can be suitably used within the range in which the effects of the present invention are not impaired.

The amount of the solvent when used is, for example, 2 to 20 parts by mass, and preferably 5 to 15 parts by mass, per 1 part by mass of the silole compound.

The present invention is preferably performed under anhydrous conditions and in an inert gas atmosphere (e.g., nitrogen gas or argon gas). The reaction temperature is, for example, 50 to 200°C, preferably 80 to 150°C, and more preferably 90 to 130°C.

After the reaction is stopped, the GNR of the present invention can be collected from the reaction liquid by an ordinary method, such as concentration, crystallization, or filtration. If necessary, purification may be performed by, for example, removal of metal by silica gel column chromatography, or separation or fractionation of polymer by gel permeation chromatography (GPC)).

### (2) GNR of the Present Invention

The GNR of the present invention has a structure represented by the following formula (1): 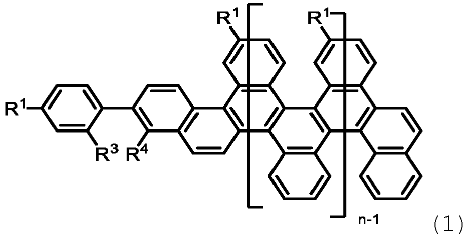 wherein R¹ represents a linear alkyl group having 1 to 12 carbon atoms, R³ and R⁴ are both hydrogen atoms, or R³ and R⁴ taken together form a group represented by -SiR^{2a}R^{2b}-, wherein R^{2a} and R^{2b} are the same or different, and each represents a hydrogen atom, an optionally branched alkyl group having 1 to 4 carbon atoms, or a phenyl group, and n represents an integer of 1 or more.

The GNR of the present invention is produced by the method for producing a GNR of the present invention. It is presumed that, by this production method, a GNR (polymer) having a structure represented by formula (1-1): 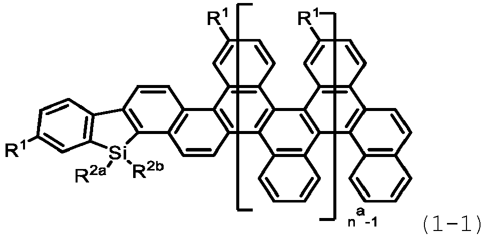
wherein R¹ represents a linear alkyl group having 1 to 12 carbon atoms, R^{2a} and R^{2b} are the same or different, and each represents a hydrogen atom, an optionally branched alkyl group having 1 to 4 carbon atoms, or a phenyl group, and n^{a} represents an integer of 1 or more,
is first formed, and that the polymer then partially or entirely undergoes a desilylation reaction to form a GNR (polymer) having a structure represented by the following formula (1-2): 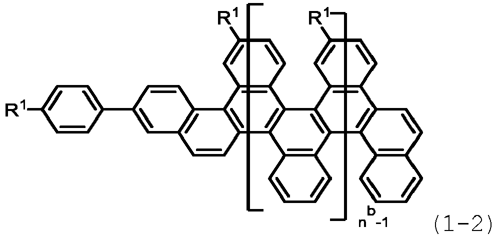 wherein R¹ represents a linear alkyl group having 1 to 12 carbon atoms, and n^{b} represents an integer of 1 or more.

In view of the above, the GNR according to the present invention may be described as comprising a GNR (polymer) represented by formula (1-1) and/or (1-2) above.

Substituents R¹, R^{2a}, and R^{2b} in formulas (1), (1-1), and (1-2) above each correspond to substituents R¹, R^{2a}, and R^{2b} in the silole compound described above; thus, the types and preferred specific examples of the substituents are also the same. According to the production method of the present invention described above, when multiple types of the silole compounds are used in combination, GNRs with different substituents R¹ can be produced. Further, according to the production method of the present invention, the GNR of the present invention can also comprise a polymer other than the GNRs (polymers) represented by formulas (1), (1-1), and (1-2) above.

The method for producing a GNR of the present invention also enables production of a GNR with a relatively higher molecular weight. The number of repeating units in the GNR of the present invention (i.e., polymerization degree n in formula (1), polymerization degree n^{a} in formula (1-1), and polymerization degree n^{b} in formula (1-2)) may be any value and can be suitably selected according to the required properties. For example, the number of repeating units can be 1 to 1000, preferably 3 to 500, and more preferably 5 to 100. The number of repeating units n, n^{a}, and n^{b} can be calculated from the number average molecular weight (Mn) measured in terms of polystyrene by SEC under the conditions described in the Examples section below.

The GNR of the present invention has a weight average molecular weight (Mw) measured in terms of polystyrene by SEC under the conditions described in the Examples section below of, for example, 2000 or more, preferably 3000 to 50000, and more preferably 4000 to 20000.

The GNR of the present invention does not contain any unit derived from an initiator compound, and such a GNR could not be produced by previously known methods. Since the GNR of the present invention has a further unified structure accordingly, the GNR is expected to have improved characteristics or develop different characteristics, compared to previously known GNRs.

The GNRs of the present invention may be ring-condensed, if necessary, by previously known methods (e.g., an oxidation reaction or a Scholl reaction) to form ring-condensed GNRs (armchair GNRs described in previously known articles). This method can be performed, for example, in accordance with the method described in PTL 1.

### Examples

The present invention is more specifically described below with reference to Examples etc. However, the present invention is not limited in any way to these Examples.

### (1) NMR Measurement

¹H-NMR and ¹³C-NMR were recorded using tetramethylsilane as an internal standard and deuterated chloroform (CDCl₃) as a solvent with JEOL ESC-600 (¹H 600 MHz, ¹³C 150 MHz) or JEOL ESC-400 (¹H 400 MHz, ¹³C 100 MHz) spectrometer. The data are described as follows.

Chemical shift, multiplicity (s = singlet, d = doublet, dd = doublet of doublets, t = triplet, sext = sextet, m = multiplet), coupling constant (Hz), and integration.

### (2) Measurement of Molecular Weights (Weight Average Molecular Weight (Mw), Number Average Molecular Weight (Mn)) of GNR Using Size Exclusion Chromatography (SEC)

The following apparatus was used for analysis under the following conditions.
Apparatus: Acquity (Advanced Polymer Chromatography)
Column: Acquity APC XT 125, 2.5 µm, 4.6×150 mm, produced by
Waters; Acquity APC XT 200, 2.5 µm, 4.6×150 mm, produced by Waters
Measurement temperature: 40°C
Solvent: Tetrahydrofuran
Molecular weight standard: Standard polystyrene

### Example 1: Synthesis Example of Silole Compound Represented by Formula (3-1)

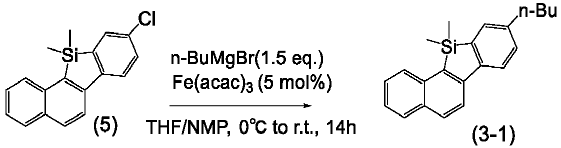

In the formula, n-Bu represents an n-butyl group, acac represents an acetylacetonate group, and THF represents tetrahydrofuran.

In a nitrogen atmosphere, the compound represented by formula (5) (1.475 g, 5.0 mmol), tris(acetylacetonato)iron(III) (Fe(acac)₃, 44.5 mg, 0.25 mmol), tetrahydrofuran (THF, 5.5 mL), and N-methyl-2-pyrrolidone (NMP, 3.4 mL) were added to a 20-mL round-bottom flask containing a magnetic stirrer. The reaction mixture was cooled to 0°C, and n-butylmagnesium bromide (1.0 M in THF, 7.5 mL, 7.5 mmol) was added at 0°C. The mixture was then heated to room temperature and stirred at room temperature for 14 hours.

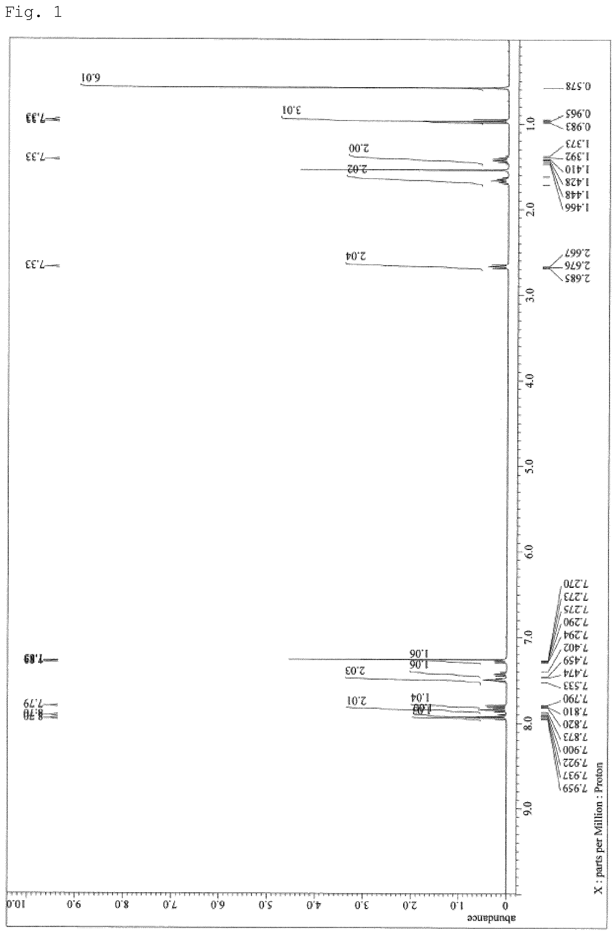

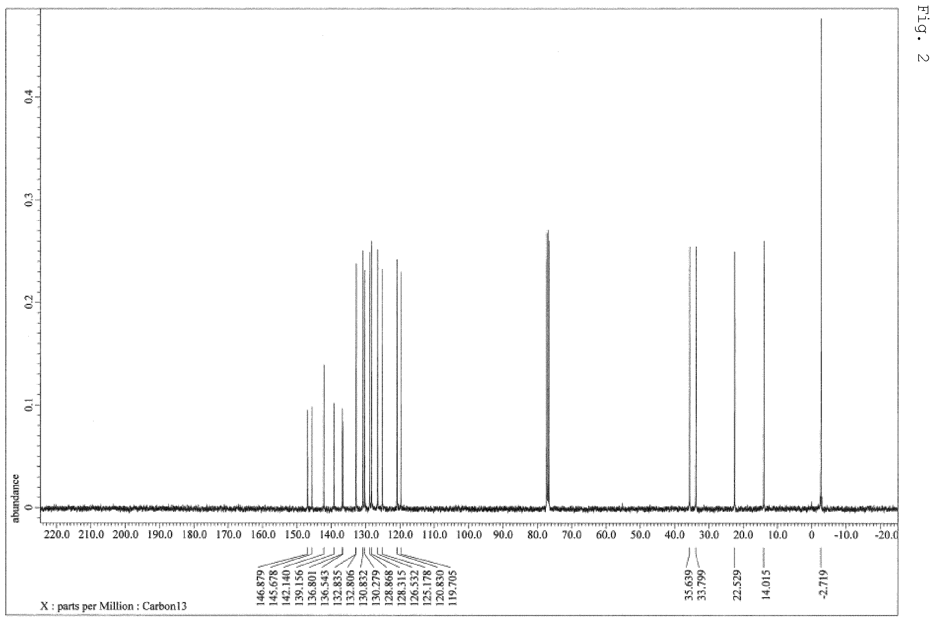

Subsequently, water was added to quench the reaction, ethyl acetate (15 mL) was added, and organic matter was extracted. After this extraction process was repeated three times in total, the organic layers were gathered and washed with saturated brine. Then, Na₂SO₄ was added and left it for overnight to dry. After drying, Na₂SO₄ was removed by filtration, and the solvent was removed under reduced pressure, and a crude product was obtained. The obtained crude product was purified by chromatography on silica gel (eluent: hexane), thereby 11,11-dimethyl-9-butyl-11H-benzo[b]naphtho[2,1-d]silole (a compound represented by formula (3-1)) was obtained as colorless crystals (535 mg, 340). The results of ¹H-NMR and ¹³C-NMR analysis of the obtained compound represented by formula (3-1) were as follows. Figs. 1 and 2 show the ¹H-NMR and ¹³C-NMR spectra.
¹H-NMR (400 MHz, CDCl₃) δ 7.95 (d, J = 8.7 Hz, 1H), 7.91 (d, J = 8.7 Hz, 1H), 7.87-7.82 (m, 2H), 7.80 (d, J = 7.8 Hz, 1H), 7.53-7.47 (m, 2H), 7.46-7.40 m, 1H), 7.28 (dd, J = 1.8, 7.8 Hz, 1H), 2.68 (t, J = 7.3 Hz, 2H), 1.71-1.62 (m, 2H), 1.42 (sext, J = 7.3 Hz, 2H), 0.97 (t, J = 7.3 Hz, 3H), 0.58 (s, 6H).
¹³C-NMR (100 MHz, CDCl₃) δ 146.9, 145.7, 142.1, 139.2, 136.8, 136.5, 132.84, 132.81, 130.8, 130.3, 128.9, 128.3, 126.5, 125.2, 120.8, 119.7, 35.6, 33.8, 22.5, 14.0, -2.7 (2C).

### Examples 2 to 6 and Comparative Examples 1 to 5: Production Examples of GNR

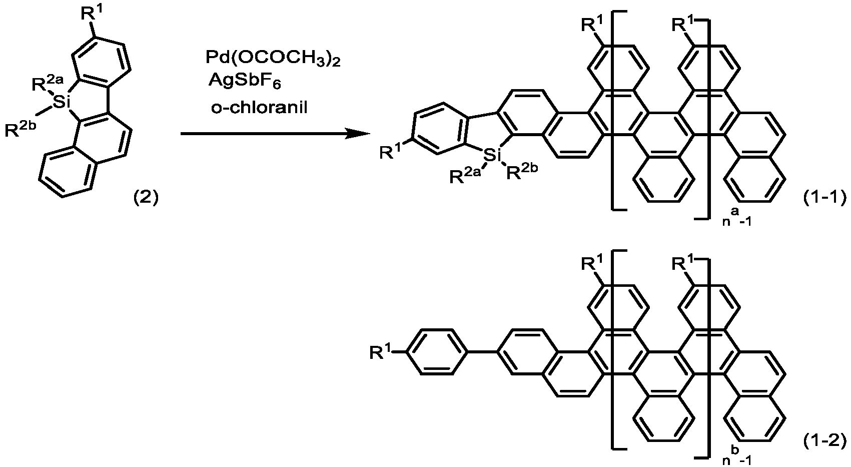

### Example 2: Production Example of GNR Represented by Formula (1) in which R¹ = n-butyl group

In a nitrogen atmosphere, a monomer (the compound represented by formula (3-1), i.e., in formula (2) above, R¹ = an n-butyl group, and R^{2a} = R^{2b} = a methyl group) (500 mg, 1.58 mmol), AgSbF₆ (108.6 mg, 0.316 mmol), Pd(OCOCH₃)₂ (70.9 mg, 0.316 mmol), o-chloranil (777 mg, 3.159 mmol), and 1,2-dichlorobenzene (3.5 mL) were added to a 20-mL Schlenk tube containing a magnetic stirrer. The mixture was then heated to 120°C and stirred at 120°C for 40 hours, thereby obtaining a reaction mixture.

The obtained reaction mixture was cooled to room temperature and passed through a short pad column of silica gel and metal scavenger by using CH₂Cl₂. The solvent was then removed under reduced pressure, and the resulting product was suspended by adding methanol, followed by filtration and drying, to thus obtain a GNR.

Analysis of the obtained GNR by size exclusion chromatography (SEC) found that Mn= 4,118, Mw= 7,385, and Mw/Mn (PDI) = 1.79.

### Examples 3 to 5 and Comparative Examples 1 to 4

GNRs were obtained in the same manner as in Example 2, except that the monomer was changed to a compound represented by formula (2) above in which R¹ = the substituent shown in Table 1 below, and R^{2a} = R^{2b} = a methyl group. Table 1 below shows the results of SEC analysis of the obtained GNRs.

**Table 1**

| | | | Molecular weight | |
|---|---|---|---|---|
| | | Substituent R¹ | Mw | Mn |
| Example | 2 | n-Bu | 7385 | 4118 |
| | 3 | Me | 4379 | 2380 |
| | 4 | Et | 5200 | 2669 |
| | 5 | n-octyl | 5209 | 3126 |
| Comparative Example | 1 | H | 1522 | 1253 |
| | 2 | tert-Bu | 1982 | 1462 |
| | 3 | Cl3* | 1486 | 1216 |
| | 4 | Cl | 2251 | 1555 |

(In the table, n-Bu represents a normal butyl group, Me represents a methyl group, Et represents an ethyl group, n-octyl represents a normal octyl group, and tert-Bu represents a tertiary butyl group. Further, Cl3* represents a substituent having the following structure: 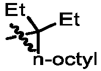 wherein n-octyl represents a normal octyl group, and the wavy line represents a bonding point.)

### Example 6

A GNR was obtained in the same manner as in Example 2, except that the amount of Pd(OCOCH₃)₂ used was changed to 0.158 mmol (0.1 equivalents based on the monomer (the compound represented by formula (3-1))), and that the amount of AgSbF₆ used was changed to 0.158 mmol (0.1 equivalents based on the monomer (the compound represented by formula (3-1))). Table 2 below shows the results of SEC analysis of the obtained GNR.

### Comparative Example 5

A GNR was obtained in the same manner as in Example 2, except that the amount of Pd(OCOCH₃)₂ used was changed to 0.79 mmol (0.5 equivalents based on the monomer (the compound represented by formula (3-1))), and that the amount of AgSbF₆ used was changed to 0.79 mmol (0.5 equivalents based on the monomer (the compound represented by formula (3-1))). Table 2 below shows the results of SEC analysis of the obtained GNR.

**Table 2**

| | | Amount used (vs 1 equivalent of monomer) | | Molecular weight | |
|---|---|---|---|---|---|
| | | Pd(OCOCH₃)₂ | AgSbF₆ | Mw | Mn |
| Example | 6 | 0.1 | 0.1 | 5255 | 3074 |
| Comparative Example | 5 | 0.5 | 0.5 | 2884 | 1655 |

## Claims

1. A graphene nanoribbon represented by formula (1): 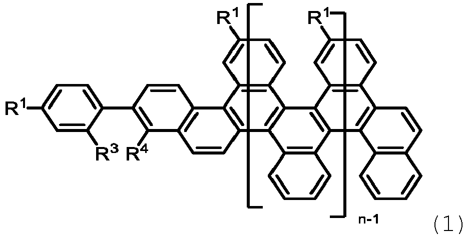 wherein R¹ represents a linear alkyl group having 1 to 12 carbon atoms, R³ and R⁴ are both hydrogen atoms, or R³ and R⁴ taken together form a group represented by -SiR^{2a}R^{2b}-, wherein R^{2a} and R^{2b} are the same or different, and each represents a hydrogen atom, an optionally branched alkyl group having 1 to 4 carbon atoms, or a phenyl group, and n represents an integer of 1 or more.

2. The graphene nanoribbon according to claim 1, which is represented by formula (1-1): 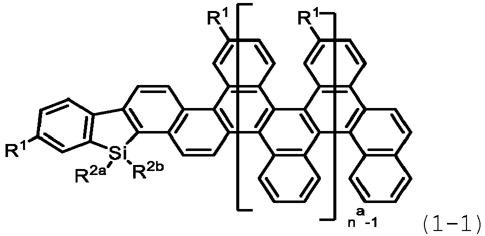
wherein R¹ represents a linear alkyl group having 1 to 12 carbon atoms, R^{2a} and R^{2b} are the same or different, and each represents a hydrogen atom, an optionally branched alkyl group having 1 to 4 carbon atoms, or a phenyl group, and n^{a} represents an integer of 1 or more; and/or
formula (1-2): 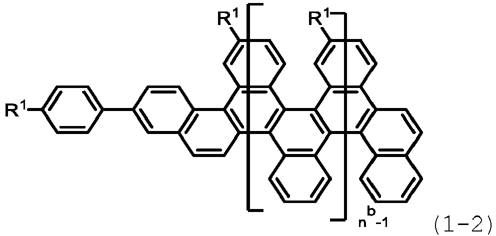
wherein R¹ represents a linear alkyl group having 1 to 12 carbon atoms, and n^{b} represents an integer of 1 or more.

3. A method for producing the graphene nanoribbon of claim 1 or 2, comprising
polymerizing a silole compound represented by formula (2): 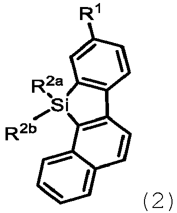
wherein R¹, R^{2a}, and R^{2b} are as defined above,
in the presence of 0.01 to 0.4 mol, based on 1 mole of silole compound represented by formula (2), of a palladium compound, o-chloranil, and a silver compound.

4. A graphene nanoribbon obtained by polymerizing a silole compound represented by formula (2): 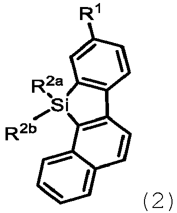
wherein R¹ represents a linear alkyl group having 1 to 12 carbon atoms, and R^{2a} and R^{2b} are the same or different, each represents a hydrogen atom, an optionally branched alkyl group having 1 to 4 carbon atoms, or a phenyl group,
in the presence of 0.01 to 0.4 mol, based on 1 mole of silole compound represented by formula (2), of a palladium compound, o-chloranil, and a silver compound.

5. A silole compound represented by formula (3): 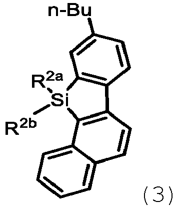 wherein n-Bu represents an n-butyl group, and R^{2a} and R^{2b} are the same or different, and each represents a hydrogen atom, an optionally branched alkyl group having 1 to 4 carbon atoms, or a phenyl group.
